# EUROPEAN PATENT APPLICATION

(11) **EP 4 424 673 A1**
(43) Date of publication of application: **04.09.2024**
(21) Application number: 22886778.4
(22) Date of filing: 17.10.2022
(51) Int. Cl.: C07D 233/56, C07C 39/15, C08G 59/62

(54) **CLATHRATE COMPOUND, EPOXY RESIN CURING AGENT, AND CURABLE RESIN COMPOSITION**

(30) Priority: 26.10.2021 JP 2021174768
(71) Applicant: ADEKA CORPORATION, Arakawa-ku Tokyo 116-8554 (JP)
(72) Inventor: KOBAYASHI, Shota, Kuki-shi, Saitama 346-0101 (JP); TAMASO, Ken-ichi, Kuki-shi, Saitama 346-0101 (JP)
(74) Representative: Forstmeyer, Dietmar
(86) International application number: PCT/JP2022/038644
(87) International publication number: WO 2023/074450

(57) **Abstract**

A clathrate compound obtained by mixing an imidazole compound (A) and a polyhydric phenol compound (B), wherein the clathrate compound has an average particle size (X) of 7 µm or less, and a maximum particle size (Y) of 40 µm or less. The imidazole compound (A) is preferably at least one selected from compounds represented by the following formula (1), 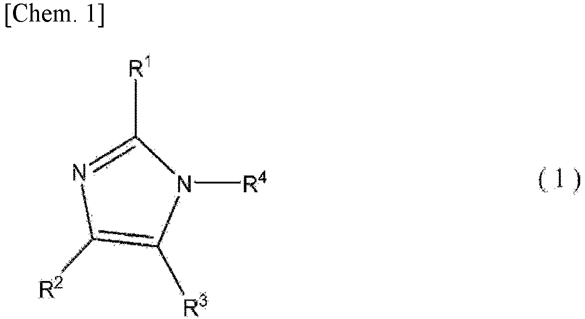 where R¹, R², R³, and R⁴ each independently represent a hydrogen atom, an alkyl group that has 1 to 20 carbon atoms and may have a substituent, or an aryl group that has 6 to 20 carbon atoms and may have a substituent, and the substituent is at least one selected from a halogen atom, a hydroxy group, and a nitrile group.

## Description

### Technical Field

The present invention relates to a clathrate compound, and specifically to a clathrate compound that is obtained by mixing an imidazole compound and a polyhydric phenol compound, and has a specific particle size, an epoxy resin curing agent that contains the clathrate compound, and a curable resin composition that contains the epoxy resin curing agent and an epoxy resin.

### Background Art

Epoxy resin is characterized as having excellent chemical resistance, corrosion resistance, mechanical properties, and thermal properties, excellent adhesiveness to various types of substrates, electric properties, usefulness in any environment, and the like, and is therefore widely used in adhesives, coating materials, electric metal materials, composite materials, and the like.

Epoxy resin compositions can be classified into one-part epoxy resin compositions and two-part epoxy resin compositions according to the type of curing agent or curing catalyst used. Two-part epoxy resin compositions have a feature of being curable at ambient temperature or low temperatures, but are also disadvantageous in that they need to be weighed and mixed immediately before use, they have a short pot life, and their conditions of use are limited as they are difficult to apply to automatic machines. Due to the aforementioned problems, one-part curable epoxy resin compositions are desirable in many applications.

Patent Literature 1 discloses that the storage stability of a one-part curable epoxy resin composition can be improved by using a clathrate compound that contains tetrakis phenol as a curing agent for epoxy resin.

### Citation List

### Patent Literature

Patent Literature 1: EP 001520867A2

### Summary of Invention

### Technical Problem

In recent years, however, demand for one-part curable epoxy resin compositions that have higher curability has continued to grow. However, the clathrate compound disclosed in Patent Literature 1 does not satisfy the demand for higher curability.

Accordingly, it is an object of the present invention to provide a curing agent for epoxy resin with which it is possible to provide a one-part curable resin composition that has higher curability than conventional ones.

### Solution to Problem

Under these circumstances, the inventors of the present application conducted in-depth studies, and found that a one-part curable curing agent for epoxy resin that has higher curability than conventional ones can be obtained by adjusting the particle size of a clathrate compound obtained by mixing an imidazole compound and a polyhydric phenol compound to a specific value or less, and arrived at the present invention.

Specifically, the present invention provides a clathrate compound obtained by mixing an imidazole compound (A) and a polyhydric phenol compound (B), wherein the clathrate compound has an average particle size (X) of 7 µm or less, and a maximum particle size (Y) of 40 µm or less.

Also, the present invention provides an epoxy resin curing agent that contains the above-described clathrate compound.

Furthermore, the present invention provides a curable resin composition that contains an epoxy resin and the above-described epoxy resin curing agent.

### Advantageous Effects of the Invention

According to the present invention, a clathrate compound obtained by mixing an imidazole compound (A) and a polyhydric phenol compound (B) has excellent storage stability and excellent curability, and it is therefore possible to provide a curing agent for epoxy resin suitable as a one-part curable epoxy resin composition. A curable resin composition obtained using the clathrate compound of the present invention has favorable stability under the presence of a solvent. The curable resin composition of the present invention is suitable for use as an adhesive for a die attach film adhesive and the like.

### Brief Description of Drawings

[Fig. 1] Fig. 1 is a ¹H-NMR chart of a clathrate compound obtained in Example 1.
[Fig. 2] Fig. 2 shows DSC curves (1°C/min) obtained in Example 3 and Comparative Example 2.
[Fig. 3] Fig. 3 shows DSC curves (1°C/min) obtained in Example 4 and Comparative Example 2.
[Fig. 4] Fig. 4 shows DSC curves (3°C/min) obtained in Example 3 and Comparative Example 2.
[Fig. 5] Fig. 5 shows DSC curves (3°C/min) obtained in Example 4 and Comparative Example 2.

### Description of Embodiments

Hereinafter, a clathrate compound according to the present invention will be described. The clathrate compound of the present invention is a clathrate compound obtained by mixing an imidazole compound (A) and a polyhydric phenol compound (B), wherein the clathrate compound has an average particle size (X) of 7 µm or less and a maximum particle size (Y) of 40 µm or less. In the following description, the imidazole compound (A) may also be referred to as "component (A)", and the polyhydric phenol compound (B) may also be referred to as "component (B)".

As used herein, the term "clathrate compound" refers to a compound that can be obtained from two or more chemical species that can exist alone in a stable manner, wherein one of the chemical species forms a molecular-scale space, and traps (clathrates) the other chemical species into the space to form a specific crystal structure, assuming that the other chemical species have a shape and dimensions with which the chemical species can be fitted into the space. The chemical species that provides the space is referred to as a "host", and the clathrated chemical species is referred to as a "guest". The host and the guest are held together by an interaction of hydrogen bonding, Van der Waals forces, or ionic bonding. It is considered that an ionic bonding clathrate compound forms an ionic crystal and a salt structure.

As the imidazole compound used in the present invention, it is preferable to use a compound represented by the following formula (1) from the viewpoint of curability and ease of forming a clathrate compound. 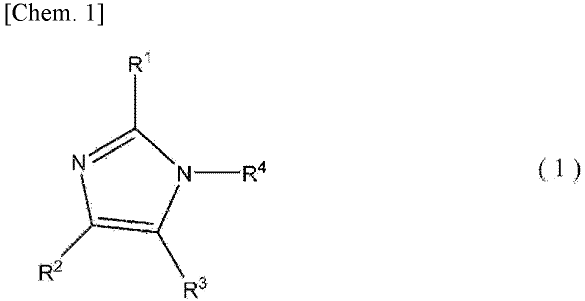 where R¹ to R⁴ each independently represent a hydrogen atom, an alkyl group that has 1 to 20 carbon atoms and may have a substituent, or an aryl group that has 6 to 20 carbon atoms and may have a substituent. The substituent may be a halogen atom or a nitrile group.

In the formula given above, examples of the alkyl group having 1 to 20 carbon atoms represented by R¹ to R⁴ include a methyl group, an ethyl group, a propyl group, an isopropyl group, a butyl group, an isobutyl group, a tertiary butyl group, a pentyl group, an isopentyl group, a tertiary pentyl group, a hexyl group, an isohexyl group, an octyl group, a 2-ethylhexyl group, a tertiary octyl group, a nonyl group, a decyl group, a undecyl group, a dodecyl group, a tridecyl group, a tetradecyl group, a pentadecyl group, a hexadecyl group, a heptadecyl group, an octadecyl group, an icosanyl group, a benzyl group, a phenethyl group, and the like. These groups may be substituted by a halogen atom, a hydroxy group, a nitrile group, or the like.

In the specification of the present application, the halogen atom may be a fluorine atom, a bromine atom, a chlorine atom, or an iodine atom.

In the formula given above, examples of the aryl group having 6 to 20 carbon atoms represented by R¹ to R⁴ include a phenyl group, a naphthyl group, and the like. These groups may be substituted by an alkyl group having 1 to 10 carbon atoms, a halogen atom, a hydroxy group, a nitrile group, or the like. In the case where the aryl group is substituted by an alkyl group, it is sufficient that the number of carbon atoms including the carbon atoms of the substituent is 6 to 20.

Out of these imidazole compounds, from the viewpoint of ease of forming a clathrate compound, it is preferable to use an imidazole compound in which R¹ represents an alkyl group having 1 to 20 carbon atoms, it is more preferable to use an imidazole compound in which R¹ represents an alkyl group having 1 to 10 carbon atoms, and it is even more preferable to use an imidazole compound in which R¹ represents an alkyl group having 1 to 4 carbon atoms. It is preferable to use an imidazole compound in which R² and R³ represent a hydrogen atom or an alkyl group having 1 to 20 carbon atoms, it is more preferable to use an imidazole compound in which R² and R³ represent a hydrogen atom or an alkyl group having 1 to 10 carbon atoms, and it is even more preferable to use an imidazole compound in which R² and R³ represent a hydrogen atom or an alkyl group having 1 to 4 carbon atoms. Also, it is preferable to use an imidazole compound in which R⁴ represents a hydrogen atom. It is particularly preferable to use an imidazole compound in which, in the formula (1), R¹ represents an alkyl group having 1 to 4 carbon atoms, R² and R³ represent a hydrogen atom or an alkyl group having 1 to 4 carbon atoms, and R⁴ represents a hydrogen atom.

From the viewpoint of ease of forming a clathrate compound, it is most preferable to use 2-ethyl-4-methylimidazole.

There is no particular limitation on the polyhydric phenol compound that is the component (B) used in the present invention as long as it is possible to form a clathrate compound when mixed with an imidazole compound. Out of polyhydric phenol compounds, from the viewpoint of ease of obtaining a clathrate compound, it is preferable to use at least one selected from tetrakis phenols represented by the following formula (2). 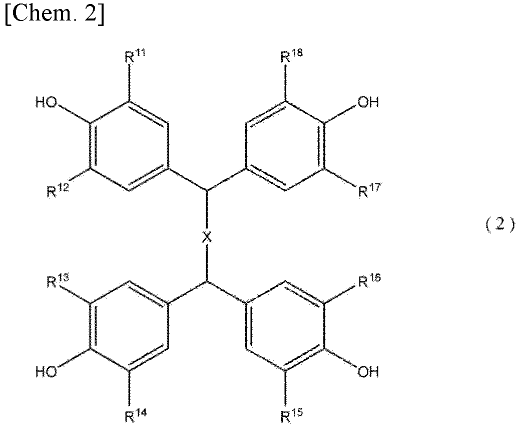 where R¹¹ to R¹⁸ each independently represent a hydrogen atom, a halogen atom, an alkyl group that has 1 to 20 carbon atoms and may have a substituent, or an aryl group that has 6 to 20 carbon atoms and may have a substituent, and X represents a single bond or a hydrocarbon group having 1 to 4 carbon atoms.

In the formula (2) given above, examples of the alkyl group having 1 to 20 carbon atoms represented by R¹¹ to R¹⁸ include a methyl group, an ethyl group, a propyl group, an isopropyl group, a butyl group, an isobutyl group, a tertiary butyl group, a pentyl group, an isopentyl group, a tertiary pentyl group, a hexyl group, an isohexyl group, an octyl group, a 2-ethylhexyl group, a tertiary octyl group, a nonyl group, a decyl group, a undecyl group, a dodecyl group, a tridecyl group, a tetradecyl group, a pentadecyl group, a hexadecyl group, a heptadecyl group, an octadecyl group, an icosanyl group, a benzyl group, a phenethyl group, and the like. These groups may be substituted by a halogen atom, a hydroxy group, a nitrile group, or the like.

In the formula (2), examples of the aryl group having 6 to 20 carbon atoms represented by R¹¹ to R¹⁸ include a phenyl group, a naphthyl group, and the like. These groups may be substituted by an alkyl group having 1 to 10 carbon atoms, a halogen atom, a hydroxy group, a nitrile group, or the like. In the case where the aryl group is substituted by an alkyl group, it is sufficient that the number of carbon atoms including the carbon atoms of the substituent is 6 to 20.

An example of the hydrocarbon group having 1 to 4 carbon atoms represented by X is an alkylene group having 1 to 4 carbon atoms. Examples of the alkylene group having 1 to 4 carbon atoms include a methylene group, an ethylene group, an ethylidene group, a n-propylene group, a propylidene group, an isopropylidene group, a methylethylene group, an n-butylene group, a butylidene group, an isobutylidene group, a sec-butylidene group, a 1,2-dimethylethylene group, a 1-methylpropylene group, a 2-methylpropylene group, and the like

Out of the compounds represented by the formula (2) given above, it is preferable to use a compound in which R¹¹ to R¹⁸ each independently represent at least one selected from a hydrogen atom, a lower alkyl group having 1 to 6 carbon atoms, and a halogen atom, because, when the obtained clathrate compound is used as a curing agent for epoxy resin, it is possible to provide a curable resin composition that has high curability. It is more preferable to use a compound in which R¹¹ to R¹⁸ each independently represent a hydrogen atom, a lower alkyl group having 1 to 3 carbon atoms, and a halogen atom. In R¹¹ to R¹⁸, the number of hydrogen atoms may be one or more, two or more, three or more, four or more, five or more, six or more, seven or more, or eight or more.

Also, X preferably represents a single bond or a hydrocarbon group having 1 to 3 carbon atoms, more preferably a single bond or a hydrocarbon group having 1 to 2 carbon atoms, and most preferably a single bond because, when the obtained clathrate compound is used as a curing agent for epoxy resin, it is possible to easily provide a curable resin composition that has high curability.

There is no particular limitation on the ratio of the component (A) and the component (B) contained in a clathrate compound as long as the clathrate compound can be formed. However, from the viewpoint of obtaining favorable curability, the amount of the compound that is the component (A) is preferably 0.1 to 10 mol, and more preferably 0.5 to 5.0 mol relative to 1 mol of the compound that is the component (B). In the case where the clathrate compound contains a component (hereinafter also referred to as a "third component") other than the component (A) and the component (B), the amount of the third component is preferably 40 mol% or less, more preferably 10 mol% or less, and even more preferably 3 mol% or less relative to the total amount of the clathrate compound. In particular, it is most preferable that the clathrate compound contains no third component. As used herein, the upper limit of the molar ratio of the third component can be applied to, in the case where the clathrate compound contains a plurality of third components, the number of moles of individual third components and the total number of moles of the third components.

There is no particular limitation on the method for mixing the clathrate compound of the present invention. However, for example, the clathrate compound can be obtained by dropping a solution obtained by dissolving the imidazole compound that is the component (A) in a solvent onto a solution obtained by dissolving the phenol compound that is the component (B) in a solvent, mixing them at a temperature from an ambient temperature to 100°C during dropping or after dropping, and then cooling and crushing the resultant. It is preferable to mix the component (A) and the component (B) while warming the component (A) and the component (B) at a temperature of 40 to 85°C.

As the solvents that can be used to dissolve the component (A) and the component (B), for example, alcoholic solvents such as methanol and 2-propanol; ester solvents such as ethyl acetate and butyl acetate; ketone solvents such as methyl ethyl ketone and acetone; aliphatic hydrocarbon solvents such as hexane, heptane, and cyclohexane; aromatic hydrocarbon solvents such as benzene, toluene, and xylene; and the like can be used. The solvent used to dissolve the component (A) and the solvent used to dissolve the component (B) may be the same solvent or different solvents. The solvent used to dissolve the component (A) and the solvent used to dissolve the component (B) may be a mixed solvent. Out of the solvents described above, it is preferable to use an ester solvent, in particular, ethyl acetate from the viewpoint of successfully producing the clathrate compound. There is no particular limitation on the concentration of the component (A) and the component (B) in the solutions in which the component (A) and the component (B) have been dissolved. However, the concentration may be, for example, 5 to 45 mass%, or 10 to 35 mass%.

As a crushing machine used in a crushing step, for example, a jet mill or the like can be used.

The clathrate compound of the present invention has an average particle size (X) of 7 µm or less and a maximum particle size (Y) of 40 µm or less. With this configuration, when the clathrate compound is used as an epoxy resin curing agent, a favorable low-temperature curability can be obtained. The average particle size (X) of the clathrate compound is preferably 0.5 µm or more from the viewpoint of storage stability. Also, the maximum particle size (Y) of the clathrate compound is preferably 1 µm or more from the viewpoint of storage stability. From the viewpoint of low-temperature curability, the average particle size (X) is more preferably 3 µm or less. Also, from the viewpoint of low-temperature curability, the maximum particle size (Y) is more preferably 10 µm or less. From these viewpoints, the average particle size (X) is even more preferably 0.5 to 3 µm. Also, the maximum particle size (Y) is even more preferably 1 to 10 µm. Furthermore, from the viewpoint of further enhancing the low-temperature curability, the ratio of the average particle size (X) relative to the maximum particle size (Y) is preferably 1:20 or less, and more preferably 1:5 or less. The ratio of the average particle size (X) relative to the maximum particle size (Y) is usually 1:1 or more, and may be 1:1.5 or more. Here, the average particle size (X) and the maximum particle size (Y) are values obtained by reading a volume-based median size (average particle size: D50, and maximum particle size: D100) using a particle size distribution measuring device based on a laser light diffraction method.

The curing agent for epoxy resin of the present invention contains the clathrate compound of the present invention as an essential component. The curing agent for epoxy resin of the present invention may contain a component other than the clathrate compound of the present invention. However, the curing agent for epoxy resin of the present invention preferably contains 30 mass% or more, and more preferably 80 mass% or more of the clathrate compound of the present invention.

The curable resin composition of the present invention contains an epoxy resin and the curing agent for epoxy resin that contains the clathrate compound of the present invention as an essential component. The amount of the clathrate compound of the present invention added to 100 parts by mass of the epoxy resin is preferably, for example, 5 parts by mass or more and 40 parts by mass or less, and more preferably 10 parts by mass or more and 30 parts by mass or less.

The epoxy resin refers to a resin that has at least two epoxy groups in a molecule, and can be used regardless of molecular structure, molecular weight, and the like.

Specific examples of the epoxy resin include: polyglycidyl ether compounds of mononuclear polyhydric phenol compounds such as hydroquinone, resorcin, pyrocatechol, and phloroglucinol; polyglycidyl ether compounds of polynuclear polyhydric phenol compounds such as dihydroxynaphthalene, biphenol, methylene bisphenol (bisphenol F), methylene bis(orthocresol), ethylidene bisphenol, isopropylidene bisphenol (bisphenol A), isopropylidene bis(orthocresol), tetrabromobisphenol A, 1,3-bis(4-hydroxycumylbenzene), 1,4-bis(4-hydroxycumylbenzene), 1,1,3-tris(4-hydroxyphenyl)butane, 1,1,2,2-tetra(4-hydroxyphenyl)ethane, thiobisphenol, sulfobisphenol, oxybisphenol, phenol novolac, orthocresol novolac, ethylphenol novolac, butylphenol novolac, octylphenol novolac, resorcin novolac, and terpene phenol; polyglycidyl ether compounds of polyhydric alcohol compounds such as ethylene glycol, propylene glycol, butylene glycol, hexanediol, polyethylene glycol, polypropylene glycol, thioglycol, dicyclopentadiene dimethanol, 2,2-bis(4-hydroxycyclohexy) propane (hydrogenated bisphenol A), glycerin, trimethylolpropane, pentaerythritol, sorbitol, and a bisphenol A-alkylene oxide adduct; homopolymers or copolymers of glycidyl ester compounds and glycidyl methacrylates of aliphatic, aromatic or alicyclic polybasic acids such as maleic acid, fumaric acid, itaconic acid, succinic acid, glutaric acid, suberic acid, adipic acid, azelaic acid, sebacic acid, dimer acid, trimer acid, phthalic acid, isophthalic acid, terephthalic acid, trimellitic acid, trimesic acid, pyromellitic acid, tetrahydrophthalic acid, and endomethylene tetrahydrophthalic acid; epoxy compounds having a glycidyl amino group such as N,N-diglycidyl aniline, bis(4-(N-methyl-N-glycidyl amino)phenyl)methane, diglycidyl orthotoluidine, N,N-bis(2,3-epoxypropyl)-4-(2,3-epoxypropoxy)-2-methylaniline, N,N-bis(2,3-epoxypropyl)-4-(2,3-epoxypropoxy)aniline, and N,N,N' ,N' -tetra(2,3-epoxy propyl)-4,4-diaminodiphenylmethane; epoxides of cyclic olefin compounds such as vinylcyclohexene diepoxide, cyclopentanediene diepoxide, 3,4-epoxycyclohexymethyl-3,4-epoxy cyclohexane carboxylate, 3,4-epoxy-6-methylcyclohexymethyl-6-methylcyclohexane carboxylate, and bis(3,4-epoxy-6-methylcyclohexymethyl)adipate; epoxidized conjugated diene polymers such as epoxidized polybutadiene, and epoxidized styrene-butadiene copolymers; and heterocyclic compounds such as triglycidyl isocyanurate. Also, the epoxy resins listed above may be internally cross-linked with a prepolymer of a terminal isocyanate, or modified with a polyvalent active hydrogen compound (a polyhydric phenol, a polyamine, a carbonyl group-containing compound, a polyphosphate ester, or the like) to have a high molecular weight. The epoxy resins listed above may be used alone or in a combination of two or more.

The amount of the epoxy resin is preferably 20 to 97 mass%, more preferably 30 to 94 mass%, and may be 40 mass% or more, or 50 mass% or more relative to the total amount of the component other than the solvents of the curable resin composition.

In the curable resin composition of the present invention, a commonly known epoxy resin curing agent may also be used together with the curing agent for epoxy resin. Examples of the commonly known epoxy resin curing agent include an anhydride curing agent, a phenol curing agent, an amine curing agent, a polythiol curing agent, and the like.

Examples of the anhydride curing agent include a himic anhydride, a phthalic anhydride, a maleic anhydride, a methyl himic anhydride, a succinic anhydride, a tetrahydrophthalic anhydride, a hexahydrophthalic anhydride, a methyltetrahydrophthalic anhydride, a methylhexahydrophthalic anhydride, a trialkyl tetrahydrophthalic anhydride-maleic anhydride adduct, a benzophenonetetracarboxylic anhydride, a trimellitic anhydride, a pyromellitic anhydride, and a hydrogenated methylnadic anhydride, and the like.

Examples of the phenol curing agent include polyhydric phenol compounds such as phenol novolac resin, cresol novolac resin, aromatic hydrocarbon formaldehyde resin modified phenol resin, dicyclopentadiene phenol-addition type resin, phenol aralkyl resin (Zyrock resin), naphthol aralkyl resin, trisphenylol methane resin, tetraphenylol ethane resin, naphthol novolac resin, naphthol-phenol co-condensation novolac resin, naphthol-cresol co-condensation novolac resin, biphenyl modified phenol resin (a polyhydric phenol compound in which a phenol nucleus is linked with a bismethylene group), biphenyl modified naphthol resin (a polyvalent naphthol compound in which a phenol nucleus is linked with a bismethylene group), aminotriazine modified phenol resin (a compound that has a phenol skeleton, a triazine ring, and a primary amino group in the molecular structure), and alkoxy group-containing aromatic ring modified novolac resin (a polyhydric phenol compound in which a phenol nucleus and an alkoxy group-containing aromatic ring are linked with formaldehyde).

Examples of the amine curing agent include: alkylene diamines such as ethylenediamine, 1,2-diamino propane, 1,3-diamino propane, 1,3-diaminobutane, 1,4-diaminobutane, hexamethylene diamine, and meta-xylenediamine; polyalkylpolyamines such as diethylenetriamine, triethylenetriamine, and tetraethylenepentamine; alicyclic polyamines such as 1,4-diaminocyclohexane, 1,3-diaminocyclohexane, 1,3-diaminomethylcyclohexane, 1,2-diaminocyclohexane, 1,4-diamino-3,6-diethylcyclohexane, 4,4' -diaminodicyclohexylmethane, 1,3-bis(aminomethyl)cyclohexane, 1,4-bis(aminomethyl)cyclohexane, 4,4'-diaminodicyclohexyl propane, bis(4-aminocyclohexyl)sulfone, 4,4'-diaminodicyclohexylether, 2,2'-dimethyl-4,4'-diaminodicyclohexylmethane, isophorone diamine, and norbornene diamine; aromatic polyamines such as diaminodiphenylmethane, diaminodiphenylsulfone, diethyltoluenediamine, 1-methyl-3,5-diethyl-2,4-diaminebenzene, 1-methyl-3,5-diethyl-2,6-diaminobenzene, 1,3,5-triethyl-2,6-diaminobenzene, 3,3'-diethyl-4,4'-diaminodiphenylmethane, and 3,5,3',5'-tetramethyl-4,4'-diaminodiphenylmethane; N,N-dimethylaminoethylamine, N,N-diethylaminoethylamine, N,N-diisopropylaminoethylamine, N,N-diallylaminoethylamine, N,N-benzylmethylaminoethylamine, N,N-dibenzylaminoethylamine, N,N-cyclohexylmethylaminoethylamine, N,N-dicyclohexylaminoethylamine, N-(2-aminoethyl)pyrrolidine, N-(2-aminoethyl)piperidine, N-(2-aminoethyl)morpholine, N-(2-aminoethyl)piperazine, N-(2-aminoethyl)-N'-methylpiperazine, N,N-dimethylaminopropylamine, N,N-diethylaminopropylamine, N,N-diisopropylaminopropylamine, N,N-diallyl aminopropylamine, N,N-benzylmethylaminopropylamine, N,N-dibenzylaminopropylamine, N,N-cyclohexylmethylaminopropylamine, N,N-dicyclohexylaminopropylamine, N-(3-aminopropyl)pyrrolidine, N-(3-aminopropyl)piperidine, N-(3-aminopropyl)morpholine, N-(3-aminopropyl)piperazine, N-(3-aminopropyl)-N'-methylpiperidine, 4-(N,N-dimethylamino)benzylamine, 4-(N,N-diethylamino)benzylamine, 4-(N,N-diisopropylamino)benzylamine, N,N,-dimethylisophorone diamine, N,N-dimethyl bisaminocyclohexane, N,N,N'-trimethylethylenediamine, N'-ethyl-N,N-dimethylethylenediamine, N,N,N'-trimethylethylenediamine, N'-ethyl-N,N-dimethyl propane diamine, N'-ethyl-N,N-dibenzylaminopropylamine; N,N-(bisaminopropyl)-N-methylamine, N,N-bisaminopropylethylamine, N,N-bisaminopropylpropylamine, N,N-bisaminopropylbutylamine, N,N-bisaminopropylpentylamine, N,N-bisaminopropylhexylamine, N,N-bisaminopropyl-2-ethylhexylamine, N,N-bisaminopropylcyclohexyamine, N,N-bisaminopropylbenzylamine, N,N-bisaminopropylallylamine, bis[3-(N,N-dimethylaminopropyl)]amine, bis[3-(N,N-diethylaminopropyl)]amine, bis[3-(N,N-diisopropylaminopropyl)]amine, bis[3-(N,N-dibutylaminopropyl)]amine, and the like; dibasic acid dihydrazides such as oxalic acid dihydrazide, malonic acid dihydrazide, succinic acid dihydrazide, glutaric acid dihydrazide, adipic acid dihydrazide, suberic acid dihydrazide, azelaic acid dihydrazide, sebacic acid dihydrazide, and phthalic acid dihydrazide; guanidine compounds such as dicyandiamide, benzoguanamine, and acetoguanamine; melamine; 2-methylimidazole, 2-ethyl-4-methylimidazole, 2-isopropylimidazole, 2-undecyl imidazole, 2-heptadecyl imidazole, 2-phenylimidazole, 2-phenyl-4-methylimidazole, 2-aminopropylimidazole, 1-phenylmethyl-2-imidazole, 1-phenylmethyl-2-ethyl-4-methylimidazole, 1-phenylmethyl-2-phenylimidazole, 1-butoxycarbonylethyl-2-methylimidazole, 1-butoxycarbonylethyl-2-ethyl-4-methylimidazole, 1-butoxycarbonylethyl-2-phenylimidazole, 1-(2-ethylhexyl)carbonylethyl-2-methylimidazole, 1-(2-ethylhexyl)carbonylethyl-2-ethyl-4-methylimidazole, 1-(2-ethylhexyl)carbonylethyl-2-phenylimidazole, 1-octyloxy carbonylethyl-2-methylimidazole, 1-octyloxy carbonylethyl-2-ethyl-4-methylimidazole, 1-octyloxy carbonylethyl-2-phenylimidazole, hexanediol • bis(2-methylimidazolyl ethanoic acid)ester, hexanediol • bis(2-ethyl-4-methylimidazolyl ethanoic acid)ester, hexanediol • bis(2-phenylimidazolyl ethanoic acid)ester, decanediol • bis(2-methylimidazolyl ethanoic acid)ester, decanediol • bis(2-ethyl-4-methylimidazolyl ethanoic acid)ester, decanediol • bis(2-phenylimidazolyl ethanoic acid)ester, tricyclopentanedimethanol • bis(2-methylimidazolyl ethanoic acid)ester, tricyclopentanedimethanol • bis(2-ethyl-4-methylimidazolyl ethanoic acid)ester, tricyclopentanedimethanol • bis(2-phenylimidazolyl ethanoic acid)ester, 1-(2-hydroxynaphthylmethyl)-2-methylimidazole, 1-(2-hydroxynaphthylmethyl)-2-ethyl-4-methylimidazole, 1-(2-hydroxynaphthylmethyl)-2-phenylimidazole, imidazole silane (for example, 2MUSIZ available from Shikoku Kasei Holdings Corporation); and the like.

It is also possible to use modified products of the amines listed above. Examples of the modification method include dehydration condensation using carboxylic acid, an addition reaction with an epoxy compound, an addition reaction with an isocyanate compound, Michael addition reaction, Mannich reaction, a condensation reaction with urea, a condensation reaction with ketone, and the like.

Examples of the carboxylic acid that can be used in the modified amine curing agent include aliphatic, aromatic or alicyclic polybasic acids such as maleic acid, fumaric acid, itaconic acid, succinic acid, glutaric acid, suberic acid, adipic acid, azelaic acid, sebacic acid, dimer acid, trimer acid, phthalic acid, isophthalic acid, terephthalic acid, trimellitic acid, trimesic acid, pyromellitic acid, tetrahydrophthalic acid, hexahydrophthalic acid, and endomethylene tetrahydrophthalic acid.

Examples of the epoxy compound that can be used in the modified amine curing agent include polyglycidyl ether compounds of mononuclear polyhydric phenol compounds such as hydroquinone, resorcin, pyrocatechol, and phloroglucinol; polyglycidyl ether compounds of polynuclear polyhydric phenol compounds such as dihydroxynaphthalene, biphenol, methylene bisphenol (bisphenol F), methylene bis(orthocresol), ethylidene bisphenol, isopropylidene bisphenol (bisphenol A), isopropylidene bis(orthocresol), tetrabromobisphenol A, 1,3-bis(4-hydroxycumylbenzene), 1,4-bis(4-hydroxycumylbenzene), 1,1,3-tris(4-hydroxyphenyl)butane, 1,1,2,2-tetra(4-hydroxyphenyl)ethane, thiobisphenol, sulfonyl bisphenol, oxybisphenol, phenol novolac, orthocresol novolac, ethylphenol novolac, butylphenol novolac, octylphenol novolac, resorcin novolac, and terpene phenol; polyglycidyl ethers of polyhydric alcohols such as ethylene glycol, propylene glycol, butylene glycol, hexanediol, polyglycol, thiodiglycol, glycerin, trimethylolpropane, pentaerythritol, sorbitol, and a bisphenol A-alkylene oxide adduct; glycidyl esters of aliphatic, aromatic, or alicyclic polybasic acids and homopolymers or copolymers of glycidyl methacrylate, the aliphatic, aromatic, or alicyclic polybasic acids including maleic acid, fumaric acid, itaconic acid, succinic acid, glutaric acid, suberic acid, adipic acid, azelaic acid, sebacic acid, dimer acid, trimer acid, phthalic acid, isophthalic acid, terephthalic acid, trimellitic acid, trimesic acid, pyromellitic acid, tetrahydrophthalic acid, hexahydrophthalic acid, and endomethylene tetrahydrophthalic acid; epoxy compounds having a glycidyl amino group such as N,N-diglycidyl aniline, bis(4-(N-methyl-N-glycidyl amino)phenyl)methane, and diglycidyl orthotoluidine; epoxides of cyclic olefin compounds such as vinylcyclohexene diepoxide, dicyclopentanediene diepoxide, 3,4-epoxycyclohexymethyl-3,4-epoxy cyclohexane carboxylate, 3,4-epoxy-6-methylcyclohexymethyl-6-methylcyclohexane carboxylate, and bis(3,4-epoxy-6-methylcyclohexymethyl)adipate; epoxidized conjugated diene polymers such as epoxidized polybutadiene, epoxidized styrene-butadiene copolymer, and heterocyclic compounds such as triglycidyl isocyanurate.

Examples of the isocyanate compound that can be used in the modified amine curing agent include: aromatic diisocyanates such as 2,4-tolylene diisocyanate, 2,6-tolylene diisocyanate, diphenylmethane-4,4'-diisocyanate, phenylene diisocyanate, xylylene diisocyanate, tetramethylxylylene diisocyanate, 1,5-naphthalene diisocyanate, 1,5-tetrahydronaphthalene diisocyanate, 3,3'-dimethyldiphenyl-4,4'-diisocyanate, dianisidine diisocyanate, and tetramethylxylylene diisocyanate; alicyclic diisocyanates such as isophorone diisocyanate, dicyclohexylmethane-4,4'-diisocyanate, trans-1,4-cyclohexyldiisocyanate, norbornene diisocyanate; aliphatic diisocyanates such as tetramethylene diisocyanate, 1,6-hexamethylene diisocyanate, 2,2,4 and/or (2,4,4)-trimethylhexamethylene diisocyanate, and lysine diisocyanate; isocyanuratetrimer compounds, buret trimer compounds, and trimethylolpropane adduct compounds of diisocyanates listed above, and the like; and triphenylmethane triisocyanate, 1-methylbenzol-2,4,6-triisocyanate , dimethyltriphenylmethanetetraisocyanate, and the like.

Furthermore, these isocyanate compounds may be used by being carbodiimide modified, isocyanurate modified, biuret modified, or the like. Alternatively, these isocyanate compounds may be used as blocked isocyanates obtained by blocking these isocyanate compounds using various types of blocking agents.

Examples of the polythiol curing agent include pentaerythritol tetrakis(3-mercaptopropionate), pentaerythritol tetrakis(thioglycolate), dipentaerythritol hexakis(3-mercaptopropionate), dipentaerythritol hexakis(3-mercaptobutyrate), 1,3,4,6-tetrakis(2-mercaptoethyl)-1,3,4,6-tetraazaoctahydropentalene-2,5-dione, 1,3,5-tris(3-mercaptopropyl)-1,3,5-triazine-2,4,6(1H,3H,SH)-trione, 4-mercaptomethyl-1,8-dimercapto-3,6-dithiaoctane, 4,8-, 4,7- or 5,7-dimercaptomethyl-1,11-dimercapto-3,6,9-trithiaundecane, and 1,3,4,6-tetrakis(2-mercaptoethyl)glycoluril. These polythiol curing agents are preferably used because excellent balance between storage stability and curability can be obtained. Preferred examples of commercially available products of the thiol compound include TS-G available from Shikoku Kasei Holdings Corporation, DPMP and PEMP available from SC Organic Chemical Co., Ltd., PETG available from Yodo Kagaku Co., Ltd., and the like.

Examples of commercially available products of the curing agent include: ADEKA HARDENER EH-3636AS (a dicyandiamide latent curing agent available from ADEKA Corporation), ADEKA HARDENER EH-4351S (a dicyandiamide latent curing agent available from ADEKA Corporation), ADEKA HARDENER EH-5011S (an imidazole latent curing agent available from ADEKA Corporation), ADEKA HARDENER EH-5046S (an imidazole latent curing agent available from ADEKA Corporation), ADEKA HARDENER EH-4357S (a polyamine latent curing agent available from ADEKA Corporation), ADEKA HARDENER EH-5057P (a polyamine latent curing agent available from ADEKA Corporation), ADEKA HARDENER EH-5057PK (a polyamine latent curing agent available from ADEKA Corporation), AJICURE PN-23 (an amine-adduct latent curing agent available from Ajinomoto Fine-Techno Co., Inc.), AJICURE PN-40 (an amine-adduct latent curing agent available from Ajinomoto Fine-Techno Co., Inc.), AJICURE VDH (a hydrazide latent curing agent available from Ajinomoto Fine-Techno Co., Inc.), Fujicure FXR-1020 (a latent curing agent available from T&K TOKA Corporation), CUREZOL (an imidazole curing agent available from Shikoku Kasei Holdings Corporation); and the like.

These may be used alone or in an appropriate combination thereof.

There is no particular limitation on the amount of the curing agent other than the clathrate compound of the present invention. However, the amount of the curing agent is preferably 10 parts by mass or more and 500 parts by mass, more preferably 100 parts by mass or more and 300 parts by mass or less, and even more preferably 30 parts by mass or less relative to 100 parts by mass of the clathrate compound of the present invention.

The curable resin composition of the present invention may optionally contain a known epoxy resin curing accelerator. Specific examples of the known epoxy resin curing accelerator include: phosphines such as triphenylphosphine; phosphonium salts such as tetraphenylphosphonium bromide; amines such as benzildimethylamine, 2,4,6-tris(dimethylaminomethyl)phenol; quaternary ammonium salts such as trimethylammonium chloride; ureas such as 3-(p-chlorophenyl)-1,1-dimethylurea, 3-(3,4-dichlorophenyl)-1,1-dimethylurea, 3-phenyl-1,1-dimethylurea, isophorone diisocyanate-dimethylurea, and tolylene diisocyanate-dimethylurea; and complex compounds obtained using boron trifluoride, amines, and ether compounds. These curing accelerators may be used alone or in a combination of two or more. There is no particular limitation on the amount of the epoxy resin curing accelerator contained in the curable resin composition of the present invention, and the amount of the epoxy resin curing accelerator can be set as appropriate according to the application of the curable resin composition.

The curable resin composition of the present invention may contain a silane coupling agent. Examples of the silane coupling agent include γ-aminopropyl triethoxy silane, N-β-(aminoethyl)-γ-aminopropyl triethoxy silane, N-β-(aminoethyl)-N'-β-(aminoethyl)-γ-aminopropyl triethoxy silane, γ-anilinopropyltriethoxy silane, γ-glycidoxypropyltriethoxy silane, β-(3,4-epoxycyclohexy)ethyltriethoxy silane, vinyltriethoxy silane, N-β-(N-vinylbenzylaminoethyl)-γ-aminopropyl triethoxy silane, γ-methacryloxy propyltrimethoxy silane, γ-chloropropyltrimethoxy silane, γ-mercaptopropyltrimethoxy silane, and the like.

The curable resin composition of the present invention may further contain a filler. Examples of the filler include silica such as fused silica and crystalline silica, magnesium hydroxide, aluminum hydroxide, zinc molybdate, calcium carbonate, silicon carbonate, calcium silicate, potassium titanate, beryllia, zirconia, zircon, forsterite, steatite, spinel, mullite, titania, and the like in the form of a powder or spherical beads, as well as glass fibers, pulp fibers, synthetic fibers, ceramic fibers, and the like.

The curable resin composition of the present invention may be dissolved in various types of solvents, preferably, an organic solvent before use. Examples include: ethers such as tetrahydrofuran, 1,2-dimethoxyethane, and 1,2-diethoxyethane; alcohols such as iso- or n-butanol, iso- or n-propanol, amyl alcohol, benzyl alcohol, furfuryl alcohol, tetrahydrofurfuryl alcohol; ketons such as methyl ethyl ketone, methyl isopropyl ketone, methyl butyl ketone; aromatic hydrocarbons such as benzene, toluene, and xylene; triethylamine, pyridine, dioxane, acetonitrile; and the like.

The curable resin composition of the present invention may optionally further contain other various types of additives. As the additives, for example, commonly used additives can be used such as: antioxidants (phenol antioxidants, phosphorus antioxidants, and sulfur antioxidants); hindered amine light stabilizers; UV absorbers; phenol compounds such as biphenol; reactive diluents such as a monoalkyl glycidyl ether; non-reactive diluents (plasticizers) such as dioctyl phthalate, dibutyl phthalate, benzyl alcohol, and coal tar; reinforcing materials such as glass cloth, aramid cloth, and carbon fiber; pigments; lubricants such as candelilla wax, carnauba wax, Japan wax, Chinese wax, beeswax, lanolin, whale wax, montan wax, petroleum wax, aliphatic wax, aliphatic esters, aliphatic ethers, aromatic esters, and aromatic ethers; thickeners; thixotropic agents; antifoaming agents; rust inhibitors; colloidal silica, colloidal alumina, and the like. In the present invention, it is also possible to use an adhesive resin such as a cyanate ester resin, a xylene resin, or a petroleum resin.

The curable resin composition of the present invention has excellent curability and storage stability. Accordingly, the curable resin composition of the present invention can be used as a one-part curable resin composition. The curable resin composition of the present invention can be preferably used in applications such as, but not limited thereto, semiconductor sealing materials, laminating agents for printed wiring boards, electronic component adhesives, electronic component sealing materials, casting materials, varnishes, coating materials, structural adhesives, and fiber reinforced composite materials.

### Examples

Next, the present invention will be described in further detail based on Examples and Comparative Examples. However, the present invention is not limited thereto.

Unless otherwise stated, the percent sign "%" used in Examples and the like given below means "mass%".

### Example 1

### Production of Clathrate Compound

An ethyl acetate solution (2E4MZ solution) of 2-ethyl-4-methylimidazole was prepared by introducing 30.0 g (0.272 mol) of 2-ethyl-4-methylimidazole and 138 g of ethyl acetate, and stirring them at 40°C for 2 hours. 53.8 g (0.135 mol) of tetrakis(4-hydroxyphenyl)ethane and 138 g of ethyl acetate were introduced into a reaction vessel to obtain a solution. The 2E4MZ solution was dropped onto the obtained solution at 22 to 23°C over 15 minutes. After that, the solution was heated to 75°C over 1 hour, and mixed at 75 to 77°C for 2 hours. Then, the solution was cooled to a temperature less than 30°C over 1 hour. After that, the solution was filtered, heat-dried at 80°C, crushed using a jet mill, and then sieved using a sieve with a mesh size of 20 µm to obtain a crushed product A-1 with an average particle size of 2.52 µm and a maximum particle size of 6.54 µm.

The average particle size and the maximum particle size were obtained using Microtrac MT-3000 II (a particle size distribution measuring device available from Microtrac BEL Corporation) based on a wet measurement method (measurement solvent: hexane). As a sample, a sample amount of 0.01 g was added to 30 ml of hexane (containing 0.5% nonionic activator) as a test sample dispersing solvent, and subjected to ultrasonic treatment (160 W) for 3 minutes, and then introduced into a measuring instrument to perform a measurement.

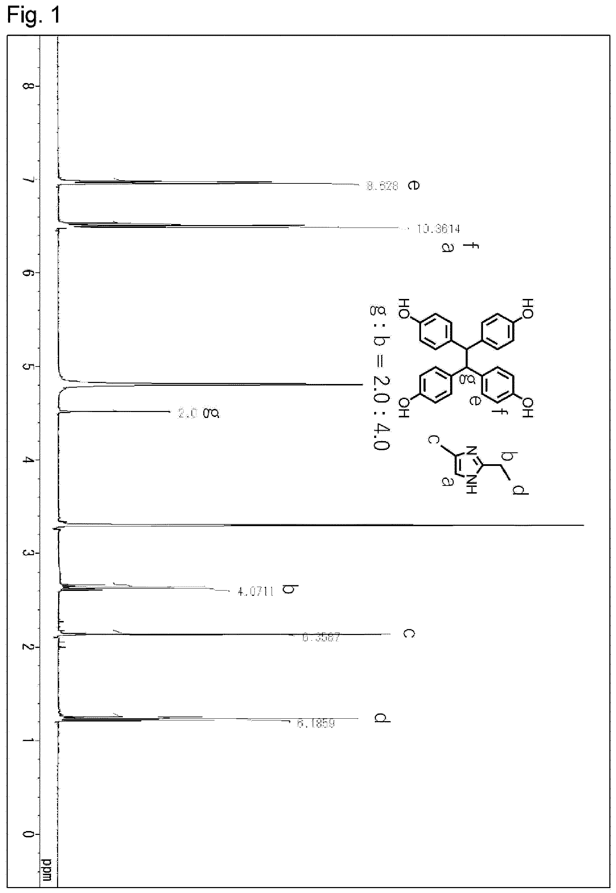

H¹-NMR data of the clathrate compound obtained is shown in Fig. 1. NMR was measured using an ECX-NMR spectrometer available from JEOL, Ltd. or an ECX-400 spectrometer available from JEOL, Ltd. As the measurement solvent, heavy DMSO was used. It had an amine number of 183 mgKOH/g. The amine number was measured using a TEAB method. As shown in Fig. 1, as a result of NMR measurement, it was confirmed that the molar ratio of tetrakis(4-hydroxyphenyl)ethane and 2-ethyl-4-methylimidazole was 1:2. In this example, it is assumed that imidazole functions as a guest, and tetrakis(4-hydroxyphenyl)ethane functions as a host.

### Example 2

### Production of Clathrate Compound

A clathrate compound (before crushing using a jet mill) obtained in the same manner as in Example 1 was crushed using a jet mill, and then sieved using a sieve with a mesh size of 10 µm to obtain a crushed product A-2 with an average particle size of 1.16 µm and a maximum particle size of 2.31 µm.

### Comparative Example 1

### Production of Clathrate Compound

A clathrate compound (before crushing using a jet mill) obtained in the same manner as in Example 1 was crushed using a jet mill to obtain a crushed product B-1 with an average particle size of 7.31 µm and a maximum particle size of 44.00 µm.

### Examples 3 and 4, and Comparative Example 2

### Curability

Samples for evaluation were prepared by mixing 11 parts by mass of each of the clathrate compounds obtained in Examples 1 and 2 and Comparative Example 1 above with 100 parts by mass of bisphenol A epoxy resin (ADEKA RESIN EP-4100E available from ADEKA Corporation).

Differential scanning calorimetry (DSC) was performed under the following conditions.

Table 1 shows average particle size, maximum particle size, peak top, and total amount of heat generation (the total amount of reaction heat generated until completion of a curing reaction when an uncured blend was used as a test sample).

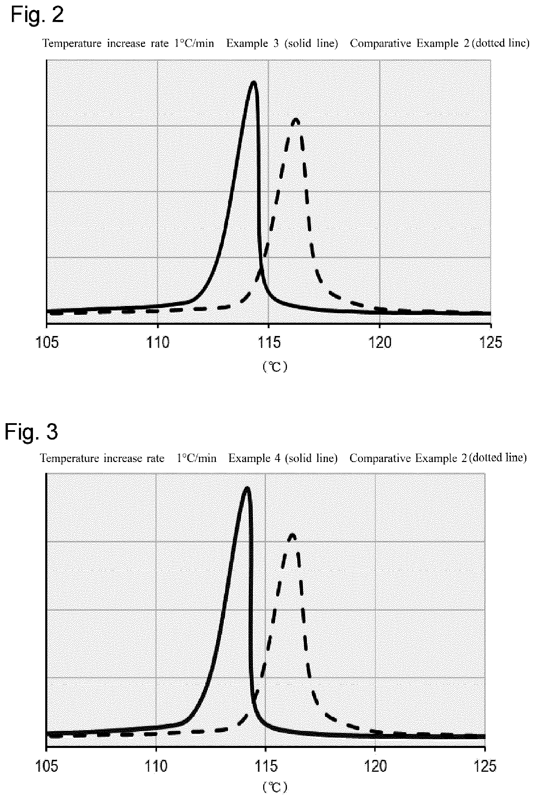

Also, Figs. 2 to 5 show portions of DSC curves obtained based on differential scanning calorimetry (DSC) near peaks.

Fig. 2 shows a result obtained when heated at a temperature increase rate of 1°C/min from 40°C to 300°C.

Fig. 3 shows a result obtained when heated at a temperature increase rate of 1°C/min from 40°C to 300°C.

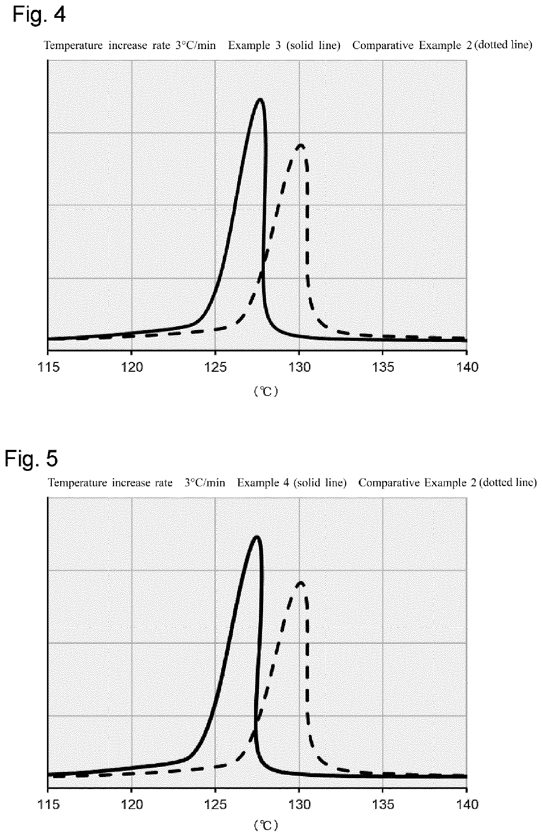

Fig. 4 shows a result obtained when heated at a temperature increase rate of 3°C/min from 40°C to 300°C.

Fig. 5 shows a result obtained when heated at a temperature increase rate of 3°C/min from 40°C to 300°C.

**[Table 1]**

| | | Example 3 | Example 4 | Comparative Example 2 |
|---|---|---|---|---|
| Clathrate compound | | A-1 | A-2 | B-1 |
| Average particle size | µm | 2.52 | 1.16 | 7.31 |
| Maximum particle size | µm | 6.54 | 2.31 | 44 |
| Peak top | °C | 114.2 / 127.7 | 114.2 / 127.5 | 116.2 / 130.0 |
| Total amount of heat generation | mJ/mg | 343 / 392 | 339 / 378 | 352 / 395 |

In colums of Peak top and Total amount of heat generation, left value was a result obtained when heated at a temperature increase rate of 1°C/min, and right value was a result obtained when heated at a temperature increase rate of 3°C/min

As shown in Table 1 given above, the clathrate compounds produced in Examples had an average particle size (X) of 7 µm or less and a maximum particle size (Y) of 40 µm or less, and thus the peak top temperature was lower than that of Comparative Example 2, and the total amount of heat generation (the amount of heat required for curing) was also effectively reduced. It is clear from this fact that the resin composition obtained using the clathrate compound of the present invention has excellent low-temperature curability.

### Example 5 and Comparative Example 3

### Stability of Curable Resin Composition Under Presence of Solvent

Bisphenol A epoxy resin (ADEKA RESIN EP-4100E available from ADEKA Corporation), the clathrate compound A-1 produced in Example 1 or 2-ethyl-4-methylimidazole, and methyl ethyl ketone were introduced into a flask in amounts shown in Table 2, and stirred and mixed with a spatula to obtain a clear solution. The amount of 2-ethyl-4-methylimidazole used in Comparative Example 3 was adjusted to be substantially the same as the amount of 2-ethyl-4-methylimidazole in the clathrate compound A-1 used in Example 5. The amount of methyl ethyl ketone was adjusted to 30 mass% in the mixture. The obtained solutions were left to stand at 25°C. Viscosity at 25°C was measured on day 0, 1, 3, and 7 after preparation using a B-type viscometer (viscosity 5 minutes after the start of measurement at a rotation speed of 60 rpm using rotor No. 1 or 2). It was not possible to measure the viscosity of the composition of Comparative Example 3 on day 7.

**Table 2]**

| | | Example 5 | Comparative Example 3 |
|---|---|---|---|
| Composition (parts by mass) | EP-4100E | 100 | 100 |
| | A-1 | 15 | - |
| | 2E4MZ | - | 5 |
| | MEK | 50 | 45 |
| B-type viscosity (mPa•s/25°C) | Dav 0 | 23.4 | 13.8 |
| | Day 1 | 15.5 | 15.5 |
| | Day 3 | 23 | 38.2 |
| | Day 7 | 1040 | Gelled |

The amount of MEK in the Table was a value that the amount of MEK in the curable resin composition was 30 mass%.

As shown in Table 2, the curable resin composition of the present invention containing 30 mass% solvent did not turn into gel and maintained low viscosity even when the curable resin composition was present together with epoxy resin for a long time.

Accordingly, it can be seen that the curable resin composition of the present invention has excellent stability under the presence of a solvent, and can also be preferably used in an application where an epoxy resin composition is required to be transformed into a varnish, for example, as a die attach film adhesive or the like.

### Industrial Applicability

According to the present invention, it is possible to provide a curable resin composition that has particularly excellent curability at low temperatures. The curable resin composition of the present invention is suitable for use as, for example, an electronic component adhesive such as a die attach film adhesive, an electronic component sealing material, a casting material, a coating material, a structural adhesive, and the like.

## Claims

1. A clathrate compound obtained by mixing an imidazole compound (A) and a polyhydric phenol compound (B),
wherein the clathrate compound has an average particle size (X) of 7 µm or less, and a maximum particle size (Y) of 40 µm or less.

2. The clathrate compound according to claim 1,
wherein the imidazole compound (A) is at least one selected from compounds represented by the following formula (1), 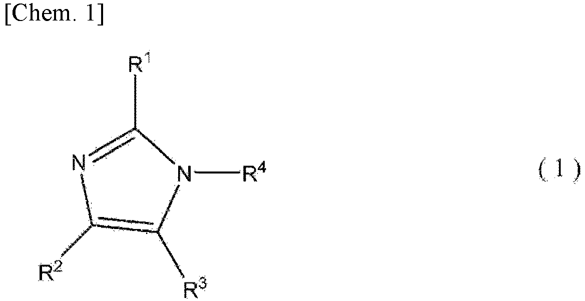 where R¹, R², R³, and R⁴ each independently represent a hydrogen atom, an alkyl group that has 1 to 20 carbon atoms and may have a substituent, or an aryl group that has 6 to 20 carbon atoms and may have a substituent, and the substituent is at least one selected from a halogen atom, a hydroxy group, and a nitrile group.

3. The clathrate compound according to claim 1 or 2,
wherein the imidazole compound (A) is 2-ethyl-4-methylimidazole.

4. The clathrate compound according to any one of claims 1 to 3,
wherein the polyhydric phenol compound (B) is at least one selected from tetrakis phenols represented by the following formula (2), 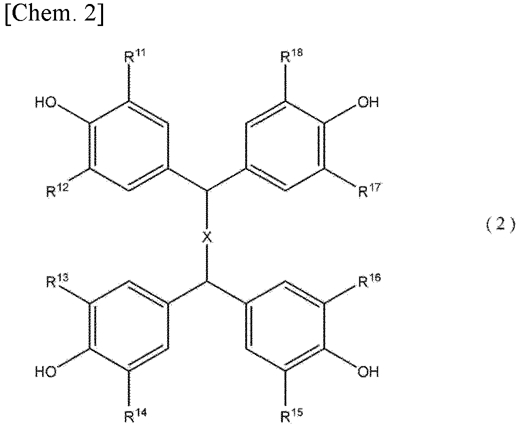 where R¹¹, R¹², R¹³, R¹⁴, R¹⁵, R¹⁶, R¹⁷, and R¹⁸ each independently represent at least one selected from a hydrogen atom, a halogen atom, an alkyl group that has 1 to 20 carbon atoms and may have a substituent, and an aryl group that has 6 to 20 carbon atoms and may have a substituent, X represents a single bond or a hydrocarbon group having 1 to 4 carbon atoms, and the substituent is at least one selected from a halogen atom, a hydroxy group, and a nitrile group.

5. The clathrate compound according to any one of claims 1 to 4,
wherein the clathrate compound has an average particle size (X) of 0.5 µm or more and a maximum particle size (Y) of 1 µm or more.

6. An epoxy resin curing agent comprising the clathrate compound according to any one of claims 1 to 5.

7. A curable resin composition comprising an epoxy resin and the epoxy resin curing agent according to claim 6.
